(19) **Europäisches Patentamt / European Patent Office / Office européen des brevets**

(11) **EP 2 143 440 A1**

(12) **DEMANDE DE BREVET EUROPEEN**

(43) Date de publication:
**13.01.2010 Bulletin 2010/02**

(51) Int Cl.:
***A61K 39/12*** *(2006.01)*

(21) Numéro de dépôt: **08305390.0**

(22) Date de dépôt: **09.07.2008**

<table>
<tr><td>

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(71) Demandeur: **Sanofi Pasteur**
**69007 Lyon Cedex (FR)**

</td><td>

(72) Inventeurs:
- **Françon, Alain**
  **69690 Bessenay (FR)**
- **Brass, Olivier**
  **69300 Caluire et Cuire (FR)**
- **Chouvenc, Pierre**
  **69009 Lyon (FR)**
- **Leleu, Amandine**
  **69007 Lyon (FR)**

</td></tr>
</table>

(54) **Agent stabilisant et composition vaccinale comprenant un ou plusieurs flavivirus vivants atténués**

(57) La présente invention concerne un agent stabilisant pour compositions vaccinales comprenant un ou plusieurs flavivirus vivants atténués, une composition vaccinale en vrac stabilisée par cet agent stabilisant, particulièrement une composition vaccinale sèche préparée à partir de cette composition vaccinale en vrac et un procédé de stabilisation d'un ou plusieurs flavivirus vivants atténués.

EP 2 143 440 A1

**Description**

La présente invention concerne un agent stabilisant pour compositions vaccinales comprenant un ou plusieurs flavivirus vivants atténués, une composition vaccinale en vrac stabilisée par cet agent stabilisant, particulièrement une composition vaccinale sèche préparée à partir de cette composition vaccinale en vrac et un procédé de stabilisation d'un ou plusieurs flavivirus vivants atténués.

Les flavivirus sont un genre de virus de la famille des flaviviridae. Ce groupe comprend le virus de la dengue (DEN), le virus de la fièvre jaune (YF), le virus de l'encéphalite de Saint Louis, le virus de l'encéphalite japonaise (JE) et le virus du Nil occidental (WN). Parmi ces virus, certains sont instables.

On entend dans le cadre de la présente invention par flavivirus tout virus de la famille des flaviviridae pathogène pour les animaux incluant les mammifères en particulier les flavivirus pathogènes pour l'homme. On peut citer à titre d'exemples non limitatifs les flavivirus suivants: les virus de la dengue sérotype 1, 2, 3 et 4 (DEN), le virus de l'encéphalite japonaise (JE), le virus de la fièvre jaune (YF), le virus du Nil occidental (WN).

Le terme vivant est utilisé dans son acception classique, un virus vivant est un virus qui n'a pas été inactivé, c'est à dire un virus capable de se répliquer sur cellules permissives.

Un flavivirus vivant atténué est un flavivirus qui n'induit pas la maladie provoquée par le virus sauvage correspondant chez l'animal ou l'homme et qui est capable d'induire une réponse immunitaire spécifique.

A titre d'exemple non limitatifs de flavivirus vivants atténués utilisables avec le stabilisant selon l'invention on peut citer les souches virales atténuées telles que la souche JEV SA-14-14-2 , la souche YF décrite dans US 6589522, les souches virales dengue décrites dans la demande WO 2006/134433 A1 (PCT/IB 2006/001313), en particulier la souche VDV1, les souches décrites dans la demande WO 2006/134443 A1 (PCT/IB 2006/001513), en particulier la souche VDV2; les souches décrites par exemple dans les demandes : WO 02/66621, WO 00/57904, WO 00/57908, WO 00/57909 ; WO 00/57910, WO 02/0950075 et WO 02/102828, les souche DEN-1 16007/PDK13, aussi appelée « LAV1 », DEN-2 16681/PDK53, aussi appelée « LAV2 », et LAV4 qui sont décrites dans la demande de brevet EP1 159 968.

La souche VDV1 est une souche obtenue à partir d'une souche sauvage DEN-1 16007 ayant subi 11 passages sur cellules PDK (DEN-1 16007/PDK11) qui a ensuite été amplifiée sur cellules Vero à 32°C, et dont l'ARN a été purifié et transfecté dans des cellules Vero. La souche VDV-1 présente 14 mutations supplémentaires par rapport à la souche vaccinale DEN-1 16007/PDK13 (13 passages sur cellules PDK-Primary Dog Kidney). La séquence complète ainsi qu'un procédé de préparation et la caractérisation de la souche VDV-1 sont donnés dans la demande PCT/IB 2006/001313. Ladite souche peut être facilement reproduite à partir de cet enseignement.

La souche VDV-2 est une souche obtenue à partir d'une souche sauvage DEN-2 16681 ayant subi 50 passages sur cellules PDK (DEN-2 16681/PDK50), purifiée par plaque et dont l'ARN a été extrait et purifié avant d'être transfecté dans des cellules Vero. La souche VDV-2 a ensuite été obtenue par purification par plaque et amplification sur cellules Vero. La souche VDV-2 présente 10 mutations supplémentaires par rapport à la souche vaccinale DEN-2 16681/PDK53 (53 passages sur cellules PDK). La séquence complète ainsi qu'un procédé de préparation et la caractérisation de la souche VDV-2 sont donnés dans la demande PCT/IB 2006/001513. Ladite souche peut être facilement reproduite à partir de cet enseignement.

Parmi des flavivirus vivants atténués utilisables avec le stabilisant selon l'invention on peut également citer - à tire non limitatif - les virus chimères tels que: les virus chimères décrits par exemple dans la demande internationale WO 93/06214 ainsi que les ChimeriVax ™. Par ChimeriVax™ ou CYD on désigne un virus de la fièvre jaune (YF) chimère qui comprend le squelette d'un virus YF dans lequel les séquences codant pour les protéines de pré-membrane et d'enveloppe ont été remplacée par les séquences correspondantes de toute souche d'un flavivirus différent, tel que par exemple, d'un virus de la dengue (DEN), d'un virus de l'encéphalite japonaise (JE), d'un virus du Nil occidentale (WN). La construction de ces ChimeriVax™ a été décrite en détails dans les demandes de brevet internationales WO 98/37911 et WO 03/101397 auxquelles on peut se reporter pour un descriptif précis de leur procédé de préparation. Une revue sur les flavivirus vivants atténués chimères utilisables dans le cadre de la présente invention donnent C.-J. Lai et T.P. Monath dans "Advances in Virus Research, (2003) vol. 61, pages 469 à 509.

On appelle ainsi CYD-1 ou CYD DEN1 un virus YF chimère contenant les séquences prM et E d'une souche dengue sérotype 1(DEN-1). On appelle CYD-2 ou CYD DEN2 un virus YF chimère contenant les séquences prM et E d'une souche DEN-2. On appelle CYD-3 ou CYD DEN3 un virus YF chimère contenant les séquences prM et E d'une souche DEN-3. On appelle CYD-4 ou CYD DEN4 un virus YF chimère contenant les séquences prM et E d'une souche DEN-4. Un virus chimère dengue peut être par exemple ChimeriVax™ DEN-1, en particulier un virus YF17D/DEN-1, ou encore un ChimeriVax™ DEN-2, en particulier un virus YF17D/DEN-2, un ChimeriVax™ DEN-3, en particulier un virus YF17D/DEN-3 ou un ChimeriVax™ DEN-4, en particulier un virus YF17D/DEN-4. Les chimères décrits dans les exemples ont été générés par utilisation des séquences prM et E issues des souches DEN 1 PUO359 (TYP1140), DEN2 PUO218, DEN3 PaH881/88 et DEN 4 1288 (TVP 980).

De préférence, le virus YF chimère comprend le squelette d'une souche de la fièvre jaune atténuée YF17D (Theiler M, and Smith HH (1937) J Exp. Med 65, p767-786.) (virus YF17D/DEN-1, YF17D/DEN-2, YF17D/DEN-3, YF17D/DEN-4). Des exemples de souches YF17D susceptibles d'être utilisées incluent YF17D204 (YF-Vax®, Sanofi-Pasteur, Swifwater, PA, USA ; Stamaril®, Sanofi-Pasteur, Marcy l'Etoile, France ; ARILVAX™, Chiron, Speke, Liverpool, UK ; FLAVIMUN®, Berna Biotech, Bern, Switzerland ; YF17D-204 France (X15067,X15062) ; YF17D-204,234 US (Rice et al., 1985, Science, 229 :726-733), ou encore des souches apparentées YF17DD (Genbank numéro d'accès U17066), YF17D-213 (Genbank numéro d'accès U17067) et les souches YF17DD décrites par Galler et al. (1998, Vaccines 16 (9/10) :1024-1028). Toute autre souche de virus de la fièvre jaune atténuée utilisable chez l'homme peut être utilisée pour la construction des chimères. Ces mêmes souches fièvre jaune peuvent être utilisées en tant que telles avec le stabilisant selon l'invention pour la préparation d'une composition utilisable dans le cadre d'une immunisation contre la fièvre jaune.

Les chimères dans lesquels le vecteur est un virus dengue, tels que décrits par exemple dans les demandes de brevets WO 96/40933 et WO 01/60847 peuvent également être utilisés avec le stabilisant selon l'invention.

Des modes de réalisation différents de virus chimères sont aussi décrits dans les demandes WO 02/102828; WO 03/103571; WO 02/072835; WO 2004/045529; WO 2005/082020, lesquels peuvent aussi être utilisés avec le stabilisant selon la présente invention.

D'autre part les vecteurs chimères décrits ci-dessus dans lesquels les séquences hétérologues insérées sont des séquences telles que décrites dans la demande WO 03/102166 peuvent également être utilisés dans le cadre de l'invention.

Les virus chimères ont la particularité de présenter les caractéristiques des virus vivants atténués tels que définis ci-dessus. On peut donc utiliser dans le cadre de l'invention tout virus chimère exprimant la protéine d'enveloppe ou un pour plusieurs épitopes d'une ou de protéine(s) d'enveloppe d'un ou de plusieurs flavivirus et induisant une réponse immune spécifique comprenant des anticorps neutralisant la souche, ou au moins l'une des souches, dont est issue la protéine d'enveloppe ou ledit épitope.

Dans le cadre de la présente invention on entend par une composition vaccinale en vrac une composition qui sort de la dernière étape de la production des antigènes, purifiée ou non, monovalente ou, après mélange multivalente.

On entend dans le cadre de la présente invention par une composition vaccinale sèche une composition, dont l'humidité résiduelle est inférieure ou égale à 3 %, prête à être reconstituée avec une solution aqueuse afin d'être utilisée en tant que vaccin ou directement sous forme sèche particulaire.

Sous le terme "spray-freeze-drying" on comprend l'atomisation d'un fluide dans un environnement cryogénique, suivi d'une lyophilisation des particules congelées obtenues.

Sous le terme "foam-drying" on comprend le séchage sous forme de mousse vitreuse par évaporation de l'eau d'une solution concentrée.

Sous le terme "freeze-foam-drying" on comprend le séchage sous forme de mousse vitreuse par sublimation de la glace d'une solution préalablement congelée, à une température au-dessus de la température de transition vitreuse et de la température d'effondrement (collapse) de la matrice.

Les compositions aqueuses de flavivirus ne permettent pas une bonne stabilité virale à long terme et à une température au dessus de 5°C. A titre d'exemple, les compositions aqueuses en vrac de la chimère YF-DEN (fièvre jaune-dengue) perdent plus de 4 log, stabilisés en liquide après stockage pendant 1 jour à 37°C. Or, cette thermolabilité représente un grave problème dans les pays YF-endémiques subtropicaux où le transport en conditions d'une chaine du froid est difficile.

Dans les années 1970 - 1980 les vaccins fièvre jaune lyophilisés présentaient un grave problème de thermo-stabilité (conservation: 6 mois à +5°C). Ces vaccins lyophilisés sans stabilisant se dégradent très rapidement lorsqu'ils sont exposés à une température en dessus de 20°C. Des efforts ont été fait pour améliorer la stabilisation des compositions vaccinales de la fièvre jaune lyophilisées (perte en 7 jours à 37 °C vaccin non stabilisé: 0,87 log, vaccin stabilisé: 0,24 log). Le stabilisant développé pour la fièvre jaune (M. Barme et C. Bronnert, J. Biol. Standardization, 1984, 12, p. 435), utilisé pour le vaccin chimère YF-DEN (fièvre jaune-dengue) se montre inefficace (perte en 7 jours à 37 °C pour le sérotype 1: 2,1 log; pour le sérotype 2: 1,3 log; pour le sérotype 3: 1,4 log; pour le sérotype 4: 1,9 log). Plus récemment, A.A. Adebayo et al. (Biologicals (1998) 26, 309-316) ont proposé des stabilisants pour un vaccin 17D fièvre jaune lyophilisé et ils constatent une perte de virus 17D vivant et efficace de 50% (0,3 log) après un stockage de 28 jours à 37°C.

La présente invention permet de résoudre le problème de la stabilisation des compositions vaccinales des flavivirus, particulièrement des vaccins des flavivirus chimères, notamment de la chimère YF-DEN (fièvre jaune-dengue).

La présente invention concerne donc un agent stabilisant pour compositions vaccinales comprenant un ou plusieurs flavivirus vivants atténués, **caractérisé en ce qu**'il comprend dans une solution aqueuse sans protéines d'origine animale et sans sels rajoutés ayant des cations divalents,

un tampon,

2,5 à 6,5% de sorbitol

2,5 à 13% de saccharose

0 à 7,5% de tréhalose et/ou 0 à 7,5% de tout autre di- ou tri-saccharide

0,2 à 0,5% d'urée

0,8 à 2,5% d'un mélange d'acides aminés comprenant arginine (Arg), cystine (Cys-Cys), histidine (His), isoleucine (Ile), leucine (Leu), lysine (Lys), méthionine (Met), phénylalanine (Phe), thréonine (Thr), tryptophane (Trp), tyrosine (Tyr), valine (Val), alanine (Ala), asparagine Asn, acide aspartique (Asp), acide glutamique (Glu), glycine (Gly), proline (Pro) et sérine (Ser).

L'agent stabilisant selon la présente invention peut contenir un ou plusieurs tampons choisis du groupe comprenant TRIS (Tris-(hydroxyméthyl)-amino-méthane), HEPES (acide 2-(4-(2-Hydroxyéthyl)-1-pipérazinyl)-éthane-sulfonique) et phosphate de potassium et/ou de sodium, le TRIS par exemple en une concentration de 5 à 10mM, le HEPES par exemple en une concentration de 7,5 à 20 mM.

Plus spécifiquement l'agent stabilisant selon la présente invention comprend 3,8 % (w/v) de sorbitol,

7,5 % (w/v) de saccharose,

5,5 % (w/v) de tréhalose,

0,25 % (w/v) d'urée et

1,5 % (w/v) de mélange d'acides aminés.

La présente invention concerne également une composition vaccinale aqueuse en vrac stabilisée qui comprend un ou plusieurs flavivirus vivants atténués et l'agent stabilisant décrit ci dessus selon l'invention.

La composition selon la présente invention peut comprendre un ou plusieurs sérotypes des virus vivants atténués de la dengue (DEN) et/ou des virus vivants atténués de la fièvre jaune (YF) et/ou des virus vivants atténués de la maladie du virus du Nil occidentale (WN) et/ou des virus vivants atténués de l'encéphalite japonaise (JE).

Des variations de la présente invention comprennent un ou plusieurs flavivirus vivants atténués chimères, par exemple un ou plusieurs sérotypes d'un virus chimère YF-DEN (fièvre jaune-dengue), d'un virus chimère YF-WN (fièvre jaune- virus du Nile occidentale) et/ou d'un virus chimère YF-JE (fièvre jaune-encéphalite japonaise).

La présente invention concerne également un procédé de stabilisation d'un ou plusieurs flavivirus vivants atténués. A la dernière étape de la production des flavivirus vivants atténués (par exemple culture sur cellules Vero, infection et culture virale suivie d'une purification à une ou plusieurs étapes) on dilue la récolte virale purifiée ou non et concentrée ou non comprenant un flavivirus vivant atténué par ajout de stabilisant de façon à obtenir les concentrations finales du stabilisant selon la présente invention afin d'obtenir une composition vaccinale aqueuse en vrac stabilisée selon la présente invention. On obtient des compositions multivalentes en mélangeant des récoltes virales purifiées ou non, concentrées ou non et stabilisées.

Le procédé de stabilisation selon la présente invention peut aussi comprendre le séchage de la composition aqueuse par une méthode sélectionnée du groupe du foam-drying, spray drying ou du freeze-foam drying, par exemple le séchage de la composition aqueuse par la méthode de la lyophilisation ou par la méthode du spray-freeze-drying. En choisissant la méthode de la lyophilisation ou du spray-freeze-drying, le procédé de stabilisation selon la présente invention peut être modifié par la congélation de la solution aqueuse en forme de particules régulières ou de billes dans une première étape et par le séchage des particules régulières ou de billes congelés dans une seconde étape afin d'obtenir un produit sec stabilisé en forme de particules régulières ou de billes. La génération de billes peut être préférablement réalisée en condition stérile. On génère les particules régulières ou les billes (ou plus concrètement les microbilles) par congélation de gouttes de la composition aqueuse selon la présente invention soit par chute dans un gaz très froid (azote liquide évaporé) soit par chute directe dans un liquide cryogénique (par exemple de l'azote liquide). Les réalisations des particules régulières ou des billes congelées à partir d'un liquide ou d'une solution aqueuse sont illustrées dans Price et al. (US 36555838), Adams et al. (US 4211015), A.A. Fawzy et al. (US 5307640), R.O. Williams III et al. (US 6862890 B2), P.F. Herbert et al. (WO 96/36317) et P.-R. Nyssen et al. (US 6903065 B2). La solution aqueuse congelée en forme de particules régulières ou de billes (microbilles) est ensuite lyophilisée. Sous le plafond à flux laminaire, les microbilles sont réparties sur des plateaux. Les étapes qui ont été réalisées sont décrites ci-dessous :

- Pré-refroidissement des étagères du lyophilisateur à -50°C

- Collage des étiquettes sur les plateaux pour l'identification des plateaux

- Refroidissement de outils de manutention et des plateaux à -50°C sur les étagères du lyophilisateur pendant une heure environ.

Les microbilles congelées sont ensuite transvasées depuis leurs conteneurs dans les plateaux. Les plateaux sont ensuite agités pour que la répartition des billes soit homogène. Les billes réparties sur le plateau sont ensuite lyophilisées. Ces particules régulières ou billes (microbilles) du produit sec stabilisé ressortant de ce procédé ont un diamètre d'environ 100 $\mu$m à 1500 $\mu$m, plus particulièrement un diamètre d'environ 500 $\mu$m à 1000 $\mu$m.

Des méthodes et appareils pour réaliser la congélation des solutions sous forme de particules régulières ou de billes congelées suivie par un séchage afin d'obtenir un produit sec sous forme de particules régulières ou de billes (microbilles) sont illustrés par exemple dans W.L. Porter et al. (US 3162019), K.M. Gover et al. (US 3431655), G.J. Malecki (US 3313032), K.D. Heck et al. (DE 26 59 546 A1), A.T.M. Wilderbeek (EP 0 799 613 A1) et D. Gehrmann et al. (US 2008/0060213 A1).

La présente invention concerne également une composition vaccinale sèche obtenue par séchage de la composition en vrac stabilisée selon la présente invention. Cette composition vaccinale sèche peut être **caractérisée en ce que** la composition est présente sous forme de particules régulières ou de billes. Cette composition vaccinale sèche sous forme de particules régulières ou de billes peut être **caractérisée en ce que** chaque particule ou chaque bille contient un mélange de différents flavivirus vivants atténués et/ou vivants atténués chimères. Cette composition vaccinale sèche sous forme de particules régulières ou de billes peut également être **caractérisée en ce que** chaque particule ou chaque bille contient un seul type de flavivirus vivants atténués et/ou vivants atténués chimères.

La présente invention concerne également un procédé de préparation d'un vaccin comprenant l'étape de reconstitution de la composition vaccinale sèche obtenue par séchage de la composition en vrac stabilisée selon la présente invention, sous forme de particules régulières ou de billes (microbilles) ou non, par une solution aqueuse.

La présente invention concerne aussi un kit d'un vaccin anti-flaviviral comprenant un premier récipient contenant la composition vaccinale sèche selon la présente invention et un second récipient contenant une solution aqueuse pour reconstituer le vaccin. Ce kit peut être **caractérisé en ce que** le premier récipient contient un mélange des différentes compositions vaccinales, chaque particule ou chaque bille contenant un seul type de flavivirus vivants atténués et/ou vivants atténués chimères.

## Exemples

**Exemple 1:** Réalisation d'une composition vaccinale aqueuse en vrac chimère fièvre jaune - dengue stabilisée

La composition monovalente a été réalisée par mélange de la chimère YF-DEN, sérotype 1, 2, 3 ou 4 (CYD-1, -2, -3 ou -4) avec le stabilisant, de façon à obtenir les concentrations en excipients stabilisants ad hoc. La composition multivalente est obtenue par mélange des compositions monovalentes.

Chaque composition vaccinale monovalente purifiée a été stabilisée avec le stabilisant de la présente invention et filtrée, homogénéisée, aliquotée puis stockée sous la forme aqueuse congelée à une température au-dessous de sa température de transition vitreuse.

Chaque composition vaccinale monovalente a été décongelée sous agitation et le volume de chaque composition vaccinale, à ajouter dans le mélange final afin de réaliser les compositions vaccinales bivalentes, trivalentes et tétravalentes dans des concentrations finales cibles a été déterminé selon le calcul suivant :

$$VolumeVrac(ml) = \frac{10^{TitreCible / dose} \times VolumePFV(ml)}{10^{TitreVrac / ml} \times VolumeRépartition / Flacon(ml)}$$

PFV signifie le Produit Final Vrac ou composition vaccinale mono- ou multivalente.

Le volume cible calculé de chaque composition vaccinale aqueuse a été prélevé et le reste du volume de composition vaccinale monovalente a été recongelé. Les volumes des compositions vaccinales monovalentes ont été mélangés avec le stabilisant de la présente invention, de manière à obtenir les concentrations finales cibles et les volumes cibles. Le mélange a été homogénéisé par agitation pendant 20 minutes à une température de 5°C et filtré de manière stérile avant la congélation ou le séchage du mélange. Le mélange avait une température de 5°C pendant les temps d'attente.

La composition vaccinale a été réalisée par mélange d'un, deux, trois, ou quatre compositions monovalentes en vrac des virus chimères fièvre jaune - dengue (Yellow Fever - Dengue, CYD) avec le stabilisant de la présente invention elles-mêmes stabilisées par mélange avec un stabilisant selon la présente invention avant la congélation ou le séchage du mélange en vrac monovalent, bivalent, trivalent et tétravalent de façon à obtenir les compositions finales dans des concentrations de cible choisie en log/ml. Les concentrations peuvent être soit équivalentes entre chaque composition vaccinale monovalente, soit différentes en fonction des cibles choisies.

**Exemple 2:** Réalisation d'une composition vaccinale lyophilisée en vrac chimère fièvre jaune - dengue stabilisée

La composition vaccinale monovalente, bivalente, trivalente ou tétravalente aqueuse a été obtenue selon la description de l'exemple 1. Ce mélange, agité et à une température de 5°C, a été réparti de manière capable et reproductible à raison de 0,3 ml par flacon pendant une durée nécessaire au chargement d'un lyophilisateur industriel. Les flacons ont ensuite été chargés sur des plateaux identifiés par des étiquettes dans le lyophilisateur, dont les étagères étaient pré-refroidies à 5°C. Les flacons comprenant la composition vaccinale aqueuse au titre cible final ont été lyophilisés suivant le cycle de lyophilisation décrit ci-après. Les flacons ont été congelés jusqu'à une température de -50°C. Une fois la température cible atteinte, la composition vaccinale congelée comprise dans chaque flacon a été sublimée pendant 16,5 h, avec une pression de 50 μbar pour la phase de sublimation primaire et une température des étagères de - 28° C. La dessiccation secondaire s'est opérée à 50 μbar et 30° C pendant 5 h. Les flacons, lyophilisés, ont ensuite été bouchés dans le lyophilisateur par pression d'étagères, sous vide ou sous pression partielle d'azote. Les flacons ont été déchargés et sertis, étiquetés, et ensuite stockés à une température de 5°C.

**Exemple 3:** Stabilité de la composition tétravalente vaccinale en vrac chimère fièvre jaune - dengue stabilisée lyophilisée, suivant l'exemple 1 et 2

La composition vaccinale tétravalente en vrac chimère fièvre jaune-dengue stabilisée aqueuse a été stabilisée avec le stabilisant de la présente invention à la concentration cible de 6 log/ml, avant de lyophiliser chaque flacon réparti à 0,3ml. Le pourcentage d'excipients de la composition vaccinale tétravalente stabilisée est indiqué dans le tableau 1 suivant :

**Tableau 1:** Pourcentage d'excipients

| EXCIPIENTS | % |
|---|---|
| Arginine L Chlorhydrate | 1,021 |
| Cystine L | 0,012 |
| Histidine L | 0,008 |
| Isoleucine L | 0,026 |
| Leucine L | 0,026 |
| Lysine L Chlohydrate | 0,037 |
| Methionine L | 0,008 |
| Phenylalanine L | 0,017 |
| Thréonine L | 0,024 |
| Tryptophane L | 0,004 |
| Tyrosine L | 0,018 |
| Valine L | 0,024 |
| Alanine L | 0,009 |
| Asparagine L | 0,015 |
| Acide L. Aspartique | 0,013 |
| Acide L. glutamique | 0,015 |
| Glycocole | 0,008 |
| Proline L | 0,012 |
| Sérine L | 0,011 |
| Saccharose | 7,500 |
| D-Tréhalose dihydrate | 5,500 |
| D-sorbitol | 3,750 |
| Tris | 0,073 |
| Urée | 0,250 |

Chaque lyophilisat a été réhydraté avant titrage avec une solution de NaCl 0,4%. 3 à 6 flacons ont été titrés pour obtenir un titre moyen.
Deux techniques de titrage ont été utilisées. La technique DICC50 est basée sur une série de dilution de la suspension virale dengue, chaque produit dilué est distribué dans les puits d'une plaque 96 puits contenant les cellules Vero. Après 7 jours de culture, les cellules Vero sont fixées et colorées lorsqu'il y a présence du virus Dengue après utilisation des anticorps monoclonaux spécifiques à chaque sérotype. Le titre infectieux est calculé selon le nombre de puits positifs, rapportés à la dilution de l'échantillon. Le DICC50 est calculé par la méthode des moindres carrés, et le titre exprimé en log10 DICC50/ml.

La technique µPFU est basée sur le même principe que la technique DICC50. Cependant, le titre est obtenu par comptage individuel des effets cytopathogènes. L'analyse se fait par analyse d'image. La précision du titrage est à +- 0,3 log.

**Tableau 2:** Résultats de stabilité du lyophilisat exprimés sous forme de perte (log)

| *Perte virale statut lyophilisat* | *log* | | | |
|---|---|---|---|---|
| | *CYD1* | *CYD2* | *CYD3* | *CYD4* |
| **perte à la lyophilisation** | 0,2 | 0,2 | 0,2 | 0,3 |
| **7 jours à 37°C** | 0,6 | 0,5 | 0,4 | 0,5 |
| **14 jours à 37°C** | 0,9 | 0,9 | 0,8 | 0,9 |
| **1 mois à 37°C** | 1,5 | 1,5 | 1,4 | 1,4 |
| **7 jours à 45°C** | 1,1 | 1,2 | 1,1 | 1,2 |
| **14 jours à 45°C** | *3,0* | *3,3* | *3,1* | *3,1* |

**Tableau 3:** Résultats de stabilité du lyophilisat réhydraté exprimés sous forme de perte (log)

| *Perte virale statut réhydraté* | *log* | | | |
|---|---|---|---|---|
| | *CYD1* | *CYD2* | *CYD3* | *CYD4* |
| **6h 5°C** | 0 | 0 | 0,1 | 0 |
| **1h 25°C** | 0,1 | 0,1 | 0,1 | 0,3 |
| **4h 25°C** | 0,3 | 0,3 | 0,3 | 0,5 |
| **2h 36°C** | *0, 4* | *0, 5* | *0, 6* | *0, 7* |

**Exemple 4:** Stabilité de la composition tétravalente vaccinale en vrac chimère fièvre jaune - dengue stabilisée lyophilisée - composition différente en sucre, suivant l'exemple 1 et 2

Le mélange saccharose 7,5%/tréhalose 5,5% a été remplacé par le saccharose à 13%, les autres composants restant inchangés.

**Tableau 4:** Résultats de stabilité du lyophilisat exprimés sous forme de perte (log)

| *Perte virale statut lyophilisat* | *log* | | | |
|---|---|---|---|---|
| | *CYD1* | *CYD2* | *CYD3* | *CYD4* |
| **perte à la lyophilisation** | 0 | 0 | 0,1 | 0,2 |
| **7 jours à 37°C** | 0,6 | 0,8 | 0,6 | 0,6 |

**Tableau 5:** Résultats de stabilité du lyophilisat réhydraté exprimés sous forme de perte (log)

| *Perte virale statut réhydraté* | *log* | | | |
|---|---|---|---|---|
| | *CYD1* | *CYD2* | *CYD3* | *CYD4* |
| **1h 25°C** | 0,8 | 0,1 | 0,4 | 0,3 |
| **4h 25°C** | 0,6 | 0,7 | 0,5 | 0,5 |

**Exemple 5:** Stabilité de la composition tétravalente vaccinale en vrac chimère fièvre jaune - dengue stabilisée lyophilisée - composition en tampon HEPES, suivant l'exemple 1 et 2

Le tampon Tris a été remplacé par le tampon HEPES, à 0,36%, les autres pourcentages de composants du stabilisant restant inchangés.

**Tableau 6:** Résultats de stabilité du lyophilisat exprimés sous forme de perte (log

| *Perte virale statut lyophilisat* | *log* | | | |
|---|---|---|---|---|
| | *CYD1* | *CYD2* | *CYD3* | *CYD4* |
| **perte à la lyophilisation** | 0,3 | 0,3 | 0,3 | 0,3 |
| **7 jours à 37°C** | 0,6 | 0,5 | 0,7 | 0,7 |
| **14 jours à 37°C** | 0,4 | 0,7 | 0,7 | 1,0 |

**Tableau 7:** Résultats de stabilité du lyophilisat réhydraté exprimés sous forme de perte (log

| *Perte virale statut réhydraté* | *log* | | | |
|---|---|---|---|---|
| | *CYD1* | *CYD2* | *CYD3* | *CYD4* |
| **1h 25°C** | 0,1 | 0,2 | 0,3 | 0,2 |
| **4h 25°C** | 0,3 | 0,1 | 0,5 | 0,5 |
| **2h 36°C** | *0,2* | *0,1* | *0,3* | *0,2* |

**Exemple 6 :** Stabilité de la composition vaccinale vrac tétravalente sous forme de microbilles lyophilisées

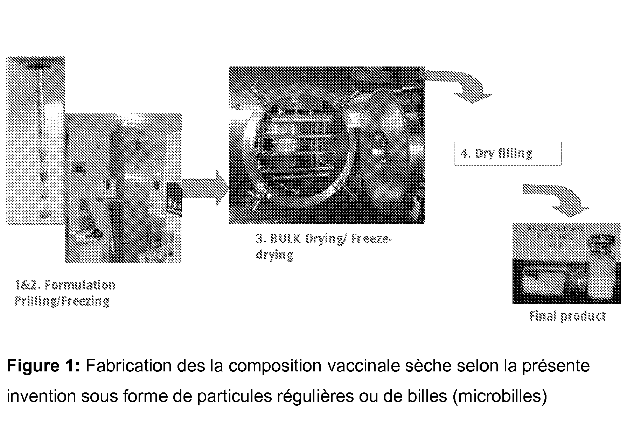

Cette étude a comparé la stabilité de la composition vaccinale vrac tétravalente, fabriquée tel que décrit dans l'exemple 1, séchée soit sous forme de lyophilisat (exemple 2) soit sous forme de microbilles lyophilisées. Les microbilles lyophilisées ont été fabriquées en utilisant le procédé illustré en figure 1 :

La composition vaccinale aqueuse en vrac a été mise sous forme de gouttelettes calibrées grâce à la technologie de « prilling » qui repose sur la vibration de buses calibrées. Ces gouttelettes ensuite congèle pendant leur chute dans une chambre cryogénique à l'intérieur de laquelle la température était maintenue en-dessous de -110°C, soit par injection directe d'azote liquide soit par balayage contre-courant de ce gaz très froid (température <-110°C). Des billes calibrées congelées ainsi obtenues ont été réparties sur des plateaux en métal préalablement refroidis. Ces plateaux ont ensuite été chargés sur les étagères pré-refroidies à -50°C d'un lyophilisateur de sorte que la température des billes congelées ne dépasse jamais leur température de transition vitreuse à cryo-concentration maximum (Tg' qui peut être situé entre - 10°C et -40°C). Ceci a permis d'éviter la fusion partielle, l'agrégation des billes ou la recristallisation de certains excipients. Une fois le produit chargé dans le lyophilisateur, l'appareil a été mis sous vide afin d'effectuer la sublimation de la glace, comme pour une lyophilisation classique du produit telle que définie par l'état de l'art. Pour cette application, les paramètres de dessiccation suivants ont été appliqués :

Dessiccation primaire à une température étagère égale à -35°C et une pression égale à 50μbar pendant 10h.

Dessiccation secondaire à une température étagère égale à 20°C et une pression égale à 50μbar pendant 3h.

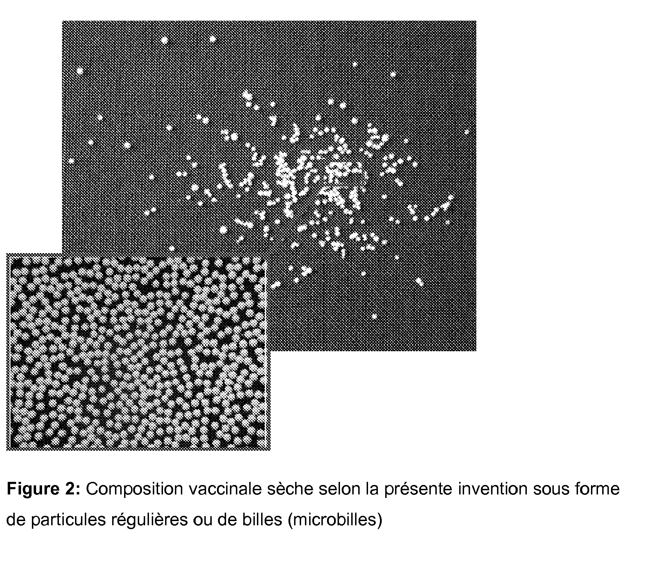

Figure 2 donne une indication sur la granulométrie des billes obtenues par ce procédé :

Les microbilles sèches ont été collectées en vrac pour être analysées et stockées. Les conditions de stockage étaient adaptées pour le stockage de poudres sèches, friables et hygroscopiques. Lors de la réhydratation des microbilles par un diluent, la reconstitution s'est instantanément faite.

L'humidité résiduelle des produits a été mesurée par Karl Fisher telle que définie par l'International Pharmacopoeia (4th Edition, Methods of Analysis: 2. Chemical methods: 2.8 Determination of water by the Karl Fischer method). La valeur obtenue était inférieure à 2%.

Les billes obtenues ont ensuite été réparties de manière à obtenir l'équivalent d'une dose par flacon. Ces flacons étaient stockés à 37°C et à 45°C afin d'étudier la thermostabilité de ce produit et de le comparer à celui séché par lyophilisation classique. L'activité des virus était dosée par la technique µPFU et les résultats obtenus sont détaillés dans les tableaux 8 à 10 ci-dessous :

**Tableau 8:** Titres initiaux pour chaque sérotype de la composition vaccinale aqueuse vrac avant séchage, exprimée en log10 PFU/dose:

|  | **CYD1** | **CYD2** | **CYD3** | **CYD4** |
|---|---|---|---|---|
| Titre d'antigène dans la composition vaccinale aqueuse vrac | 4,9 | 5,5 | 5,6 | 5,7 |

**Tableau 9:** Perte d'activité pour chaque sérotype au cours du séchage sous forme de microbilles, log10 PFU/dose:

|  | CYD1 | CYD2 | CYD3 | CYD4 |
|---|---|---|---|---|
| Perte du titre infectieux | 0.4 | 0.2 | 0.2 | 0.2 |

**Tableau 10:** Résultats de thermostabilité à 37°C et à 45°C pour chaque serotypes des microbilles lyophilisées :

| Perte du titre infectieux | PFU/dose | | | |
|---|---|---|---|---|
|  | CYD1 | CYD2 | CYD3 | CYD4 |
| 7 jours à 37°C | 0,3 | 0,3 | 0,3 | 0,3 |
| 14 jours à 37°C | 0,6 | 0,5 | 0,6 | 0,6 |
| 1 mois à 37°C | 1,2 | 1,2 | 1,2 | 1,1 |
| 7 jours à 45°C | 0,9 | 0,8 | 0,9 | 0,8 |
| 14 jours à 45°C | 2,7 | 2,4 | 2,3 | 2,2 |

Pour chacun des 4 sérotypes, les pertes au cours du procédé de séchage étaient équivalentes entre la lyophilisation (décrite dans les exemples 2 et 3) et le procédé microbille (entre 0,2 et 0,4 log10PFU/dose). La forme sèche sous forme de microbille a présenté une meilleure thermostabilité à 37°C et à 45°C que la forme lyophilisée standard avec une différence de 0,3 log après 1 mois à 37°C, différence qui s'est accentuée jusqu'à atteindre 0,8 log après 14 jours de stockage à 45°C.

Etant donné que cette différence de stabilité a été observé avec des compositions vaccinales identiques, les conditions opératoires du procédé microbilles et plus particulièrement la phase de congélation rapide a un effet bénéfique pour la stabilité des virus vivants chimériques.

**Revendications**

1. Agent stabilisant pour compositions vaccinales comprenant un ou plusieurs flavivirus vivants atténués, **caractérisé en ce qu'**il comprend dans une solution aqueuse sans protéines d'origine animale et sans sels rajoutés ayant des cations divalents,
un tampon,
2,5 à 6,5% de sorbitol
2,5 à 13% de saccharose
0 à 7,5% de tréhalose et/ou 0 à 7,5% de tout autre di- ou tri-saccharide
0,2 à 0,5% d'urée
0,8 à 2,5% d'un mélange d'acides aminés comprenant arginine (Arg), cystine (Cys-Cys), histidine (His), isoleucine (Ile), leucine (Leu), lysine (Lys), méthionine (Met), phénylalanine (Phe), thréonine (Thr), tryptophane (Trp), tyrosine (Tyr), valine (Val), alanine (Ala), asparagine Asn, acide aspartique (Asp), acide glutamique (Glu), glycine (Gly), proline (Pro) et sérine (Ser).

2. Agent stabilisant selon la revendication 1, **caractérisé en ce qu'**il comprend un ou plusieurs tampons choisis du groupe comprenant TRIS (Tris-(hydroxyméthyl)-amino-méthane), HEPES (acide 2-(4-(2-Hydroxyéthyl)-1-pipérazinyl)-éthane-sulfonique) et phosphate de potassium et/ou de sodium.

3. Agent stabilisant selon la revendication 2 **caractérisé en ce que** le TRIS est présent en une concentration de 5 à 10mM.

4. Agent stabilisant selon la revendication 2 **caractérisé en ce que** le HEPES est présent en une concentration de 7,5 à 20 mM.

5. Agent stabilisant selon l'une ou plusieurs des revendications 1 à 4 comprenant
3,8 % (w/v) de sorbitol,
7,5 % (w/v) de saccharose,
5,5 % (w/v) de tréhalose,
0,25 % (w/v) d'urée et
1,5 % (w/v) de mélange d'acides aminés.

6. Composition vaccinale aqueuse en vrac stabilisée comprenant un ou plusieurs flavivirus vivants atténués et l'agent stabilisant selon l'une ou plusieurs des revendications 1 à 5.

7. Composition selon la revendication 6, **caractérisée en ce qu'**elle comprend un ou plusieurs sérotypes des virus vivants atténués de la dengue (DEN).

8. Composition selon l'une ou plusieurs des revendications 6 ou 7, **caractérisée en ce qu'**elle comprend des virus vivants atténués de la fièvre jaune (YF).

9. Composition vaccinale selon l'une ou plusieurs des revendications 6 à 8, **caractérisée en ce qu'**elle comprend des virus vivants atténués de la maladie du virus du Nil occidentale (WN).

10. Composition vaccinale selon l'une ou plusieurs des revendications 6 à 9, **caractérisée en ce qu'**elle comprend des virus vivants atténués de l'encéphalite japonaise (JE).

11. Composition vaccinale selon l'une ou plusieurs des revendications 6 à 10, **caractérisée en ce qu'**elle comprend un ou plusieurs flavivirus vivants atténués chimères.

12. Composition vaccinale selon la revendication 11, **caractérisée en ce qu'**elle comprend un ou plusieurs sérotypes d'un virus chimère YF-DEN (fièvre jaune-dengue).

13. Composition vaccinale selon l'une ou plusieurs des revendications 11 à 12, **caractérisée en ce qu'**elle comprend d'un virus chimère YF-WN (fièvre jaune- virus du Nile occidentale).

14. Composition vaccinale selon l'une ou plusieurs des revendications 11 à 13, **caractérisée en ce qu'**elle comprend d'un virus chimère YF-JE (fièvre jaune-encéphalite japonaise).

15. Procédé de stabilisation d'un ou plusieurs flavivirus vivants atténués comprenant la dilution d'une récolte virale purifiée et concentrée comprenant un ou plusieurs flavivirus vivants atténués par ajout de stabilisant de façon à obtenir les concentrations finales du stabilisant définies selon les revendications 1 à 5 afin d'obtenir une composition selon l'une ou plusieurs des revendication 6 à 14.

16. Procédé de stabilisation selon l'une des revendications15, comprenant le séchage de la composition aqueuse par une méthode sélectionnée du groupe du foam-drying, spray drying ou du freeze-foam drying.

17. Procédé de stabilisation selon l'une des revendications 15, comprenant le séchage de la composition aqueuse par la méthode de la lyophilisation.

18. Procédé de stabilisation selon l'une des revendications 15, comprenant le séchage de la composition aqueuse par la méthode du spray-freeze-drying.

19. Procédé de stabilisation selon l'une des revendications 17 ou 18, **caractérisé en ce que,** dans une première étape, la solution aqueuse est congelée en forme de particules régulières ou de billes et que, dans une seconde étape, les particules régulières ou billes congelées sont soumises au séchage afin d'obtenir un produit sec stabilisé en forme de particules régulières ou de billes.

**20.** Procédé de stabilisation selon la revendication 19, **caractérisé en ce que** les particules régulières ou billes du produit sec stabilisé ont un diamètre d'environ 100 $\mu$m à 1500 $\mu$m.

**21.** Procédé de stabilisation selon la revendication 20, **caractérisé en ce que** les particules régulières ou billes du produit sec stabilisé ont un diamètre d'environ 500 $\mu$m à 1000 $\mu$m

**22.** Composition vaccinale sèche obtenue par séchage de la composition en vrac stabilisée selon l'une ou plusieurs des revendications 6 à 14.

**23.** Composition vaccinale sèche selon la revendication 22, **caractérisée en ce que** la composition est présente sous forme de particules régulières ou de billes.

**24.** Composition vaccinale sèche selon la revendication 23, **caractérisée en ce que** chaque particule ou chaque bille contient un mélange de différents flavivirus vivants atténués et/ou vivants atténués chimères.

**25.** Composition vaccinale sèche selon la revendication 23, **caractérisé en ce que** chaque particule ou chaque bille contient un seul type de flavivirus vivants atténués et/ou vivants atténués chimères.

**26.** Procédé de préparation d'un vaccin comprenant l'étape de reconstitution de la composition selon l'une des revendications 22 à 25 par une solution aqueuse.

**27.** Kit d'un vaccin anti-flaviviral comprenant un premier récipient contenant la composition vaccinale sèche selon l'une des revendications 22 à 25 et un second récipient contenant une solution aqueuse pour reconstituer le vaccin.

**28.** Kit selon la revendication 27 **caractérisé en ce que** le premier récipient contient un mélange des différentes compositions vaccinales selon la revendication 25.

**Figure 1:** Fabrication des la composition vaccinale sèche selon la présente invention sous forme de particules régulières ou de billes (microbilles)

**Figure 2:** Composition vaccinale sèche selon la présente invention sous forme de particules régulières ou de billes (microbilles)

Europäisches
Patentamt

European
Patent Office

Office européen
des brevets

**RAPPORT DE RECHERCHE EUROPEENNE**

Numéro de la demande

EP 08 30 5390

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | WO 2007/056847 A (SANOFI PASTEUR LTD [CA]; CIGARINI SANDRINE [CA]) 24 mai 2007 (2007-05-24) * page 2, alinéa 6 * * page 3, alinéa 8 - page 4, alinéa 11 * * page 6, alinéa 16; revendications * ----- | 1-28 | INV. A61K39/12 |
| A | EP 0 065 905 A (PASTEUR INSTITUT [FR]) 1 décembre 1982 (1982-12-01) * page 4, ligne 34 - page 6, ligne 29 * ----- | 1-28 | |
| D,A | ADEBAYO A A ET AL: "Stability of 17D Yellow Fever virus vaccine using different stabilizers" BIOLOGICALS, vol. 26, no. 4, décembre 1998 (1998-12), pages 309-316, XP002504097 ISSN: 1045-1056 * le document en entier * ----- | 1-28 | |
| A | SOOD D K ET AL: "Study on the stability of 17D-204 yellow fever vaccine before and after stabilization" VACCINE, BUTTERWORTH SCIENTIFIC. GUILDFORD, GB, vol. 11, no. 11, 1 janvier 1993 (1993-01-01), pages 1124-1128, XP023710602 ISSN: 0264-410X [extrait le 1993-01-01] * le document en entier * ----- | 1-28 | DOMAINES TECHNIQUES RECHERCHES (IPC) A61K |

Le présent rapport a été établi pour toutes les revendications

| Lieu de la recherche | Date d'achèvement de la recherche | Examinateur |
|---|---|---|
| La Haye | 14 novembre 2008 | Montero Lopez, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...............................................................................
& : membre de la même famille, document correspondant

EPO FORM 1503 03.82 (P04C02)

**EP 2 143 440 A1**

ANNEXE AU RAPPORT DE RECHERCHE EUROPEENNE
RELATIF A LA DEMANDE DE BREVET EUROPEEN NO.

EP 08 30 5390

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

14-11-2008

| Document brevet cité au rapport de recherche | Date de publication | Membre(s) de la famille de brevet(s) | Date de publication |
|---|---|---|---|
| WO 2007056847 A | 24-05-2007 | AU 2006315026 A1 | 24-05-2007 |
| | | CA 2630349 A1 | 24-05-2007 |
| | | EP 1951865 A1 | 06-08-2008 |
| | | KR 20080070866 A | 31-07-2008 |
| EP 0065905 A | 01-12-1982 | DE 3260939 D1 | 15-11-1984 |
| | | FR 2505657 A1 | 19-11-1982 |
| | | JP 1932142 C | 26-05-1995 |
| | | JP 4006689 B | 06-02-1992 |
| | | JP 58032827 A | 25-02-1983 |
| | | JP 2060806 C | 10-06-1996 |
| | | JP 6065096 A | 08-03-1994 |
| | | JP 7068143 B | 26-07-1995 |
| | | US 4500512 A | 19-02-1985 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6589522 B
- WO 2006134433 A1
- WO 2006001313 W
- WO 2006134443 A1
- WO 2006001513 W
- WO 0266621 A
- WO 0057904 A
- WO 0057908 A
- WO 0057909 A
- WO 0057910 A
- WO 020950075 A
- WO 02102828 A
- EP 1159968 A
- WO 9306214 A
- WO 9837911 A
- WO 03101397 A
- WO 9640933 A
- WO 0160847 A

- WO 03103571 A
- WO 02072835 A
- WO 2004045529 A
- WO 2005082020 A
- WO 03102166 A
- US 36555838 B, Price
- US 4211015 A, Adams
- US 5307640 A, A.A. Fawzy
- US 6862890 B2, R.O. Williams III
- WO 9636317 A, P.F. Herbert
- US 6903065 B2, P.-R. Nyssen
- US 3162019 A, W.L. Porter
- US 3431655 A, K.M. Gover
- US 3313032 A, G.J. Malecki
- DE 2659546 A1, K.D. Heck
- EP 0799613 A1, A.T.M. Wilderbeek
- US 20080060213 A1, D. Gehrmann

**Littérature non-brevet citée dans la description**

- **C.-J. Lai ; T.P. Monath.** *Advances in Virus Research,* 2003, vol. 61, 469-509
- **Theiler M ; Smith HH.** *J Exp. Med,* 1937, vol. 65, 767-786
- **Rice et al.** *Science,* 1985, vol. 229, 726-733
- **Galler et al.** *Vaccines,* 1998, vol. 16 (9/10), 1024-1028

- **M. Barme ; C. Bronnert.** *J. Biol. Standardization,* 1984, vol. 12, 435
- **A.A. Adebayo et al.** *Biologicals,* 1998, vol. 26, 309-316
- l'International Pharmacopoeia. **Karl Fisher.** Methods of Analysis. 2